# EUROPEAN PATENT SPECIFICATION

(11) **EP 2 978 842 B1**
(45) Date of publication and mention of the grant of the patent: **02.05.2018**
(21) Application number: 14718180.4
(22) Date of filing: 26.03.2014
(51) Int. Cl.: C12N 9/10

(54) **GLUCAN BRANCHING ENZYMES AND THEIR METHODS OF USE**
GLUCANVERZWEIGUNGSENZYME UND DEREN VERFAHREN ZUR VERWENDUNG
ENZYMES DE RAMIFICATION DE GLUCANE ET LEURS PROCÉDÉS D'UTILISATION

(30) Priority: 26.03.2013 US 201361805360 P
(43) Date of publication of application: 03.02.2016
(73) Proprietor: Matis ohf., 113 Reykjavik (IS)
(72) Inventor: JONSSON WHEAT, Jon Oskar, IS-112 Reykjavik (IS); HREGGVIDSSON, GudmundurOli, IS-101 Reykjavik (IS); FRIDJONSSON, Olafur Hedinn, IS-108 Reykjavik (IS); DOBRUCHOWSKA, Justyna M., NL-9747 AG Groningen (NL); KAMERLING, Johannis P., NL-9747 AG Groningen (NL)
(74) Representative: Arnason Faktor
(86) International application number: PCT/IS2014/050003
(87) International publication number: WO 2014/155395

(56) References cited:
- Jón Óskar Jónsson ET AL: "[beta]-Glucan Transferases of Family GH17 from Proteobacteria", , 1 January 2010 (2010-01-01), XP055127410, Retrieved from the Internet: URL:http://skemman.is/stream/get/1946/7050 /19017/1/Beta-Glucan_Transferases_from_Pro teobacteria.pdf [retrieved on 2014-07-08] cited in the application
- G. O. HREGGVIDSSON ET AL: "Exploring novel non-Leloir -glucosyltransferases from proteobacteria for modifying linear ( 1->3)-linked gluco-oligosaccharide chains", GLYCOBIOLOGY, vol. 21, no. 3, 1 March 2011 (2011-03-01), pages 304-328, XP055127468, ISSN: 0959-6658, DOI: 10.1093/glycob/cwq165 cited in the application
- A. GASTEBOIS ET AL: "Characterization of a New (1-3)-Glucan Branching Activity of Aspergillus fumigatus", JOURNAL OF BIOLOGICAL CHEMISTRY, vol. 285, no. 4, 22 January 2010 (2010-01-22), pages 2386-2396, XP055127415, ISSN: 0021-9258, DOI: 10.1074/jbc.M109.077545 cited in the application
- J P Kamerling: "Analysis of glycans synthesized by microbial trans-glycosylating enzymes", , 1 January 2014 (2014-01-01), XP055127811, Retrieved from the Internet: URL:http://ics27bangalore.in/abstracct-dow nload/Plant-animal-Industrial-polysacchari des/V-A-IL-1.pdf [retrieved on 2014-07-09]

### SEQUENCE LISTING

<110> MatÃ-s ohf.
   Rijksuniversiteit Groningen
<120> Glucan Branching Methods
<130> P9416PC00
<150> US61/805,360
   <151> 2013-03-26
<160> 2
<170> BiSSAP 1.0
<210> 1
   <211> 283
   <212> PRT
   <213> Bradyrhizobium diazoefficiens
<220>
   <221> SOURCE
   <222> 1..283
   <223> /mol_type="protein"
   /note="Glt20 glycosyltransferase"
   /organism="Bradyrhizobium diazoefficiens"
<400> 1
   Ala Gly Leu Trp Gly Val Leu Arg Asp Lys Glu Pro Ala Pro Asp Phe
   1               5                   10                  15
   Arg Gly Leu Leu Pro Ser Val Ser Tyr Ala Pro Phe Glu Gly Ser Ala
               20                  25                  30
   His Pro Asp Ile Asp Asn Ile Pro Thr Val Glu Lys Ile Arg Ala Asp
           35                  40                  45
   Leu Lys Thr Leu Ser Thr Met Thr Arg Ala Ile Arg Leu Tyr Ser Ser
       50                  55                  60
   Thr Gly Gly Val Glu Leu Val Pro Ala Ile Ala Ala Glu Phe Gly Leu
   65                  70                  75                  80
   Lys Val Thr Val Gly Ala Trp Ile Asp Lys Asp Lys Asp Arg Asn Glu
                   85                  90                  95
   Arg Glu Ile Lys Ala Ala Ile Glu Leu Ala Arg Lys Asn Ser Asn Val
               100                 105                 110
   Val Gly Val Val Val Gly Asn Glu Val Ile Tyr Arg Gly Glu Gln Lys
           115                 120                 125
   Val Glu Asp Leu Ile Asp Met Ile Lys Lys Val Lys Gly Ser Val Arg
       130                 135                 140
   Val Pro Val Thr Thr Gly Glu Ile Trp Asn Ile Trp Arg Asp Asn Pro
   145                 150                 155                 160
   Asp Leu Ala Ser Asn Val Asp Phe Ile Ala Ala His Val Leu Pro Tyr
                   165                 170                 175
   Trp Glu Asn Phe Arg Ser Asp Gln Ala Val Asp Gln Ala Val Asp Arg
               180                 185                 190
   Tyr Asn Leu Leu Arg Asn Leu Phe Pro Gly Lys Arg Ile Val Ile Ala
           195                 200                 205
   Glu Phe Gly Trp Pro Ser Gln Gly Tyr Asn Leu Arg Asn Ala Asp Pro
       210                 215                 220
   Gly Pro Phe Gln Gln Ala Leu Thr Leu Arg Asn Phe Val Ser Arg Ala
   225                 230                 235                 240
   Glu Ala Ile Gly Met Glu Tyr Asn Ile Val Glu Ala Ile Asp Gln Pro
                   245                 250                 255
   Trp Lys Phe Phe Glu Gly Gly Val Gly Pro Tyr Trp Gly Ile Leu Asn
               260                 265                 270
   Ala Ser Arg Glu Pro Lys Phe Ala Trp Thr Gly
           275                 280
<210> 2
   <211> 283
   <212> PRT
   <213> null
<220>
   <221> SOURCE
   <222> 1..283
   <223> /mol_type="protein" /note="glycosyltransferase" /organism=null
<400> 2
   Ala Ala Leu Trp Gly Leu Leu Glu Lys Lys Glu Thr Ala Pro Asp Phe
   1               5                   10                  15
   Arg Gly Met Leu Pro Ser Val Ser Tyr Ala Pro Phe Glu Gly Ser Ala
               20                  25                  30
   His Pro Asp Val Asp Asn Ile Pro Ser Val Glu Lys Ile Arg Asp Asp
           35                  40                  45
   Leu Arg Lys Leu Ser Lys Ile Thr Lys Ala Ile Arg Leu Tyr Ser Ser
       50                  55                  60
   Thr Gly Gly Val Glu Leu Val Pro Pro Ile Ala Ala Glu Phe Gly Leu
   65                  70                  75                  80
   Lys Val Thr Val Gly Ala Trp Leu Asp Lys His Leu Asp Arg Asn Glu
                   85                  90                  95
   Arg Glu Ile Ala Ala Ala Ile Asp Leu Ala Lys His Asn Ser Asn Val
               100                 105                 110
   Ile Ala Val Val Val Gly Asn Glu Thr Leu Tyr Arg Ala Asp Leu Lys
           115                 120                 125
   Val Asp Glu Leu Ile Asp Tyr Ile Gln Arg Val Lys Arg Gln Val Asn
       130                 135                 140
   Val Pro Val Thr Thr Gly Glu Ile Trp Ser Met Trp Arg Asp Glu Pro
   145                 150                 155                 160
   Arg Leu Ser Ser Ser Val Asp Phe Ile Ala Ala His Ile Leu Pro Tyr
                   165                 170                 175
   Trp Asn Asn Ile Pro Ala Gly Ser Ala Val Asp Tyr Ala Met Thr Ile
               180                 185                 190
   Ser Lys Leu Leu Arg Asp Ser Phe Pro Gly Lys Arg Val Val Val Ala
           195                 200                 205
   Glu Phe Gly Trp Pro Ser Gln Gly Tyr Asn Leu Lys Ser Ala Glu Pro
       210                 215                 220
   Gly Pro Phe Glu Gln Ala Ser Ile Leu Arg Asn Phe Val Thr Arg Ala
   225                 230                 235                 240
   Glu Ser Ile Gly Leu Asp Tyr Asn Ile Val Glu Ala Ile Asp Gln Pro
                   245                 250                 255
   Trp Lys Phe Phe Glu Gly Gly Val Gly Pro Tyr Trp Gly Ile Leu Asn
               260                 265                 270
   Ala Ser Arg Glu Pro Lys Phe Pro Trp Thr Gly
           275                 280

## Description

### BACKGROUND OF THE INVENTION

Glycosyltransferases catalyze the transfer of a glycosyl group from a suitable carbohydrate donor to a suitable carbohydrate acceptor. Recently, a non-Leloir β-1,3(β-1,6)-glucosyltransferase activity has been described for some enzymes in the glycosyl hydrolase family GH17 originating from fungi and bacteria (Gastebois et al., 2010; Hreggvidsson et al., 2011). The catalytic activity of these enzymes can be described as a two-step retaining mechanism. The first step involving the cleavage of a (β1→3) linkage of a β-glucan oligosaccharide or polysaccharide, such as from laminarin, lichenan, curdlan or related polysaccharides or oligosaccharides, and the second step is the formation of a (β1→6) linkage to another laminarin or related polysaccharide or oligosaccharide chain.

β-Glucans, made up of (β1→3)- or (β1→4)-linked glucopyranosyl residues, are important cell wall components of bacteria, fungi, seaweeds, and plants. They can be further classified on the basis of presence/absence of other linkages into various distinct types with different physicochemical properties. Thus, laminarin that can be extracted from seaweed has predominantly (β1→3) linkages with (β1→6)-linked side branching on every 10th glucose subunit on average. On the other hand, β-glucans from barley, oat or wheat have mixed (β1→3) and (β1→4) linkages in the backbone, but no (β1→6) branches, and generally higher molecular weights and viscosities (McIntosh et al., 2005).

A linear glucan chain has two different ends, called reducing (R-end) and non-reducing (NR-end). The glucosyl transfer reaction requires two main steps. 1) The enzyme must bind to a donor substrate molecule and cleave the glucan chain with the R-end part released and the NR-end part covalently attached to the enzyme. 2) The enzyme then must bind an acceptor glucan molecule and transfer the bound glucan chain onto the acceptor. Therefore the donor becomes smaller and the acceptor larger. In glycosyl hydrolases the acceptor is water and no addition to the acceptor occurs. Most enzymes transfer short NR-ends of distinct lengths, i.e. 1, 2, 3 or 4 glucose units. Such enzymes are therefore defined as cutting from the NR-end. Until now, all known bacterial β-glucan transferases are of this type (Hreggvidsson et al., 2011).

### The prior art on enzymes with β(1-3)-glucan branching activity

Glucosyltransferases acting on β-glucans are rare but have been described from certain yeasts and fungi (Gastebois et al., 2010). These enzymes release laminaribiose from the reducing end of a linear (β1→3)-linked oligosaccharide and transfer the remaining chain to another oligosaccharide acting as acceptor. The transfer occurs at C-6 of the non-reducing end group of the acceptor, creating a kinked (β1→3;β1→6) linear molecule or the transfer takes place at the C-6 of an internal group of the acceptor, forming a (β1→3)-linked branched product with a (β1→6) linkage. The enzyme from *Aspergillus fumigatus* was shown to make up to 85% of a branched product when incubated for less than 1 h with substrates of larger than five glucose units.

In bacteria, proteins belonging to the GH17 family were proposed, based on mutagenesis studies on the *ndvB* gene, to be involved in biosynthesis of cyclic (β1→3,β1→6)-glucans from *Bradyrhizobium japonicum* (Bhagwat et al., 1995), but no enzyme activity has been characterized before A number of bacterial putative transglycosylases belonging to glycosyl hydrolase family GH17 domains were identified, each encoded by a gene also encoding a GT2 glycosyl transferase domain in *Gamma-Proteobacteria.* The GH17 domains were subsequently cloned separately (from GT2) in *E. coli.* These recombinant enzymes named Glt1, Glt3 and Glt7, were shown to be non-Leloir β-glucan transferases. They cleave short (β1→3)-linked gluco-oligosaccharides substrates from the non-reducing end. The Glt1 and Glt3 enzymes exhibited mainly (β1→3) elongation activity, but in the case of the Glt7 enzyme also (β1→6) transfer was seen, resulting in a mixture of branched and kinked products (Hreggvidsson et al.). For Glt7 the yield of the (β1→6) transfer products was however small, since the total transfer of both (β1→3) (the major reaction) and (β1→6) was approximately 10% on free laminari-oligosaccharides but much less on alditols with a maximum incubation time of 24 h. Glt7 also had high hydrolysis activity which together with the poor transfer activity makes it unefficient glucantransferase and not suitable for any industrial use.

A further GH17 bacterial domain from the *ndvB* gene (encoding both a GH17 domain and GT2 domain) was identified in *Alpha-Proteobacterium, Bradyrhizobium japonicum* USDA110. It was cloned and expressed by the same authors (Jonsson 2010) and the enzyme product, named Glt20, shown to resemble the previously characterized *A. fumigatus* enzyme in the sense that it specifically cleaves laminaribiose from the reducing end of a linear (β1→3)-glucan and transfers the remainder to an acceptor oligosaccharide with a (β1→6) linkage. A new name *B. diazoefficiens* has recently been proposed for *B. japonicum* (Delamuta et al. 2013). Both names are therefore used here as meaning the same species and strains where appropriate. Based on the work of Jonsson (2010) the authors concluded previously that the Glt20 enzyme created a kinked (β1→3;β1→6) linear molecule but not branches.

### SUMMARY OF THE INVENTION

The present invention provides new methods which are defined in the claims, based on surprising features and newly identified and characterised branching activity of the Glt20 enzyme from *Bradyrhizobium japonicum,* in particular the GH17 domain with MalE attachment removed in the case of expression of the gene with this attachment, and taxonomically related enzymes as defined herein. The inventors have been able to ascertain that in this form the Glt20 has a (β1→6) transferase activity, that cuts by releasing two interlinked glucose units (laminaribiose) from the R-end of (β1→3)-linked gluco-oligosaccharides and therefore transfers a long NR-end to the acceptor, similar as has earlier been described for enzymes from certain fungi and yeasts (Gastebois et al., 2010). However, there are distinct differences between the Glt20 bacterial enzyme and the methods of the present invention versus the fungal enzymes and their described activity. The fungal enzyme makes (β1→6)-linked branches as the major product but also (β1→6)-linked kinks, and it has significant hydrolysis activity as well (Gastebois et al. 2010). The Glt20 bacterial enzyme makes only branches, both single and double (and even triple), and no kinks, even after 48 h incubation when substrate donor oligosaccharide is present. Only after extended incubation of more than 48 h, when the substrate is depleted, are linear (β1→6 kinks) products seen. Such linear (kinked) products are therefore only made from hydrolysis of the non-reducing end of the branched products, but not from direct transfer to the terminal non-reducing end of the acceptor. It has therefore been concluded by the inventors that the Glt20 enzyme is a glucan transferase that specifically transfers the donor oligosaccharide to C-6 of an internal group of the acceptor and makes no transfer to a C-6 on the non-reducing end of the acceptor. This activity is found to be highly efficient. The amount of the formed oligosaccharides (DP>5) from Lam-Glc5 after 48 h, including Pro-Glc₈₋₁₁₋₁₄, was estimated to be about 60% of the total initial substrate amount. When the original substrate (donor) molecule is used up, the Glt20 enzyme shows a limited hydrolysis activity on the branched products and conversion into linear (kinked) products is seen upon extended incubation. The linear kinked products are formed because of the inability of the enzyme to cleave internal 1,6-branch points made by prior transfer events. When the donor substrate is depleted and the reaction goes to completion a substantial amount of 1,6-linkages are found in the products. Using Lam-Glc5 as a substrate donor, the 1,6-linkages increased to 26% in the final products from 0% in the donor, Lam-Glc5. The Glt20 bacterial enzyme according to the present study had no activity on oligosaccharide-alditols, unlike the *A. fumigatus* fungal enzyme. The Glt20 bacterial enzyme starts branching on residue 3 (counting from the reducing end), but currently it is not known at what residue the branches are introduced by the fungal enzyme.

As the Glt20 enzyme is able to cleave the substrate from the R-end, it should be able to produce cyclic molecules by transferring the new donor R-end to the acceptor NR-end of the same molecule, if the original substrate β-glucan chain is long enough. This would be in agreement with the earlier proposed role of the *ndvB* gene (Glt20) from mutagenesis studies in *Bradyrhizobium japonicum* (Bhagwat et al., 1995). However, such cyclization has not been observed in the present work. The reported beta-glucan cycle by Bhagwat et al., (1999) has only 1,3-linkages, which is incompatible with the activity of *ndvB* GH17 enzymes as reported herein. It would be expected to close such cycles with a 1,6-linkage.

Many β-glucans are bio-active compounds, for example containing immuno-stimulating activity such as, anti-tumoral, anti-infectious, protection against fungi, bacteria and viruses infections and various health benefits to humans. The physiological activity of β-glucans is however affected by the type and degree of branching (B 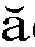 dulescu et al., 2009). Therefore, suitable glucosyltransferase enzyme activities can be used to add carbohydrate side chains onto glucans, and therefore make highly branched molecules that may be useful for many industrial or commercial applications. Enzymes having specific glucosyltransferase activity of the invention may thus have advantageous and useful applications such as for making new bio-active molecules, including bio-active molecules for cosmetic and medical applications, including molecules used for drug design such as molecules used as constituents of drugs.

The present invention therefore shows that when the Glt20 enzyme is produced, without any expressed attachments such as GT2 (native gene organization) or -purification tags such as MalE (for purification) it has unexpected and highly useful properties. The specific internal branching activity with no terminal transfer and almost no hydrolysis activity of the recombinant Glt20 as produced and utilized herein, was unexpected. The enzyme is surprisingly an efficient enzyme under *in vitro* conditions and is expected to be useful and consequently to have large potential for industrial application and commercial use.

### DESCRIPTION OF THE INVENTION

The present invention relates to the use of β-glucan branching enzymes in transglycosylation reactions, and more specifically methods for transglycosylating substrate beta-glucan oligo- and polysaccharides to produce certain useful and valuable products.

As such, the invention is based on the use of an isolated glucosyltransferase of a bacterial origin that has glucosyltransferase activity on (β1→3)-glucan oligo- and polysaccharides such that it releases two interlinked glucose units from the reducing end of such a β-glucan oligo/polysaccharide (acting as donor molecule) and transfers the remaining non-reducing end of said β-glucan oligo/polysaccharide to internal glucose units of an additional β-glucan oligo/polysaccharide (acting as acceptor) and not to the terminal glucose unit of said additional β-glucan oligo/polysaccharide, or preferentially to an internal site rather than the terminal site.

The term "isolated" as used herein means that the material is removed from its original environment (e. g. the natural environment where the material is naturally occurring). For example, a polynucleotide or polypeptide while present in a living source organism is not isolated, but the same polynucleotide or polypeptide, which is separated from some or all of the coexisting materials in the natural system, is isolated. Such polynucleotides could for example be part of a vector and/or such polynucleotides or polypeptides could be part of a composition, and still be isolated in that the vector or composition is not part of the natural environment. When referring to a particular enzyme or any polypeptide, the term "isolated" refers to a preparation of the polypeptide outside its natural source and preferably substantially free of contaminants.

Methods of producing replicate copies of the same polynucleotide, such as PCR or gene cloning, are collectively referred to herein as "amplification" or "replication". For example, single- or double-stranded DNA can be replicated to form another DNA with the same sequence. RNA can be replicated, for example, by RNA directed RNA polymerase, or by reverse transcribing the RNA and then performing a PCR. In the latter case, the amplified copy of the RNA is a DNA with the correlating or homologous sequence.

The polymerase chain reaction ("PCR") is a reaction in which replicate copies are made of a target polynucleotide using one or more primers, and a catalyst of polymerization, such as a DNA polymerase, and particularly a thermally stable polymerase enzyme. Generally, PCR involves repeatedly performing a "cycle" of three steps: 1) "melting", in which the temperature is adjusted such that the DNA dissociates to single strands, 2) "annealing", in which the temperature is adjusted such that oligonucleotide primers are permitted to anneal to their complementary nucleotide sequence to form a duplex at one end of the polynucleotide segment to be amplified; and 3) "extension" or "synthesis", which can occur at the same or slightly higher and more optimum temperature than annealing, and during which oligonucleotides that have formed a duplex are elongated with a thermostable DNA polymerase. The cycle is then repeated until the desired amount of amplified polynucleotide is obtained. Methods for PCR amplification can be found in U.S. Patents No. 4,683,195 and 4,683,202.

The methods disclosed herein involving the molecular manipulation of nucleic acids are known to those skilled in the art. See generally Ausubel, F.M. et al., "Short Protocols in Molecular Biology," John Wiley and Sons (1995); and Sambrook, J., et al., "Molecular Cloning, A Laboratory Manual," 2nd ed., Cold Spring Harbor Laboratory Press (1989).

In one embodiment of the invention, the isolated glucosyltransferase of a bacterial origin has glucosyltransferase activity such that the transferred non-reducing end of one (β1→3)-glucan oligo/polysaccharide molecule is transferred to the third glucose residue from the reducing end of a different (β1→3)-glucan oligo/polysaccharide.

In certain embodiments of the invention, the isolated glycosyltransferase of a bacterial origin is not active on oligosaccharide-alditols as substrate.

The invention also encompasses certain methods for carrying out transglycosylation reactions that involve adding a donor substrate and an acceptor substrate for transglycosylation to a reaction mixture. Then a protein comprising an enzymatically active GH17 protein domain of bacterial origin as described herein can be added to the reaction, and the reaction incubated until the transglycosylation reaction occurs and run for a desired time.

In one embodiment, the invention is a method for transglycosylation of substrate (β1→3)-glucan oligo/polysaccharides using an isolated glucosyltransferase of a bacterial origin that has glucosyltransferase activity on β-glucan oligo/polysaccharides such that it releases two interlinked glucose units from the reducing end of said β-glucan oligo/ polysaccharides and transfer the remaining non-reducing end of said β-glucan oligo/ polysaccharide to internal glucose units of an additional β-glucan oligo/polysaccharide and not to the terminal glucose unit of said additional oligo/polysaccharide.

As understood from herein, it is particularly advantageous that in useful embodiments the enzyme used in the methods has preferential transferase activity over hydrolase activity in the presence of reactive substrate β-glucan oligo- or polysaccharide that can act as acceptor. 'Preferential' as used in this context means that preferably the transferase reaction occurs at least at twofold rate of hydrolysis, and more preferably at fourfold rate, and even more preferably at least at tenfold rate, and yet more preferably at 20-fold rate, or 100-fold rate, over the hydrolysis reaction.

As mentioned, the enzymes used in the methods of the invention are of bacterial origin, selected from bacterial glycosyltransferases from the GH17 family. A definition of the term GH17 family can be found e.g. on CAZypedia.org (see http://www.cazypedia.org/index.php/Glycoside Hydrolase Family 17).

In certain embodiments the enzyme used for the transglucosylation reaction is produced recombinantly from a DNA sequence comprising a coding region for GH17 enzyme from the taxonomic family of *Bradyrhizobiaceae*, such as from the genera of *Bradyrhizobium*, *Rhodopseudomonas*, *Nitrobacter*, and *Afipia.*

Also, in certain embodiments the GH17 enzymes can be from, *B. japonicum*, *B. diazoefficiens, Nitrobacter hamburgensis, Rhodopseudomonas palustris* and *Afipia clevelandensis*, among other organisms.

The above mentioned enzymes are shown by inventors to share critical sequence similarity in particular in the regions around the substrate binding cleft. Representative embodiments of such enzymes useful in the invention are compared in Example 1.

The isolated enzyme comprises a sequence with 75% or higher sequence identity to SEQ ID NO: 1, and more preferably 85% or higher, and even more preferably 90% or higher identity, such as 95% or higher.

Algorithms for sequence comparisons and calculation of "sequence identity" are known in the art as discussed above, such as BLAST, described in Altschul et al. 1990, or the Needleman and Wunsch algorithm (Needleman and Wunsch 1970) Generally, the default settings with respect to e.g. "scoring matrix" and "gap penalty" will be used for alignment. The percentage sequence identity values referred to herein refer to values as calculated with the Needleman and Wunsch algorithm such as implemented in the program Needle (Rice et al. 2000) using the default scoring matrix EBLOSUM62 for protein sequences, (or scoring matrix EDNAFULL for nucleotide sequences) with opening gap penalty set to 10.0 and gap extension penalty set to 0.5. The sequence identity is thus the percentage of identical matches between the two sequences over the aligned region including any gaps in the length. Percentage identity between two sequences in an alignment can also be counted by hand such as the sequence identity in an alignment that has been manually adjusted after automatic alignment.

In one embodiment, the invention is a method for transglycosylation of substrate (β1→3)-glucan oligo/polysaccharides using a glucosyltransferase of a bacterial origin defined above, such that the produced branched polysaccharides have biological activity.

In one embodiment of the invention said biological activity includes immune activity such as anti-tumoral, anti-bacterial, anti-viral or anti-fungal activity. In certain methods the donor substrate in the reaction can be laminarin, lichenan, curdlan, scleroglucan and pustulan or related polysaccharides and the like or their mixtures.

In certain embodiments of the methods the acceptor substrate in the reaction can comprise oligosaccharide from laminarin, cellulose, lichenan, curdlan scleroglucan and pustulan or related polysaccharides and the like or their mixtures.

In certain methods the donor or the acceptor substrates in the reaction can be oligosaccharides or polysaccharides and the like or their mixtures.

Additional features and advantages of the present invention are described in, and will be apparent from, the following Detailed Description of the Invention and the figures.

In certain embodiments the enzyme used for the transglucosylation reaction can be made from a gene where certain DNA sequences have been added, mutated or deleted. In certain embodiments the enzyme used for the transglucosylation reaction according to the invention can be recombinantly expressed in bacteria such as E. coli or any other suitable bacterial expression system known to the skilled person. In certain other embodiments the enzyme used for the transglucosylation reaction can be expressed from eukaryotic organisms such as but not limited to yeasts or fungi. The invention also contemplates certain recombinant vectors for carrying the DNA sequences and transfecting effectively a chosen expression system such that the enzyme is recombinantly expressed.

The enzymes used in the methods of the invention can be partially or substantially purified (e.g., purified to homogeneity), and/or are substantially free of other polypeptides. According to the invention, the amino acid sequence of the enzyme can be that of the naturally occurring enzyme or can comprise alterations therein. Polypeptides comprising alterations are referred to herein as "derivatives" of the native polypeptide. Such alterations include conservative or non-conservative amino acid substitutions, additions and deletions of one or more amino acids; however, such alterations should preserve the transglycosylation activity of the enzyme, *i.e*., the altered or mutant polypeptides of the invention are active derivatives of the naturally occurring polypeptide having the specific transglycosylation activity. Preferably, the amino acid substitutions are of minor nature, i.e. conservative amino acid substitutions that do not significantly alter the folding or activity of the polypeptide. Deletions are preferably small deletions, typically of one to 30 amino acids. Additions are preferably small amino- or carboxy-terminal extensions, such as amino-terminal methionine residue; a small linker peptide of up to about 25 residues; or a small extension that facilitates purification by changing net charge or another function, such as a poly-histidine tail, an antigenic epitope or a binding domain. The alteration(s) should preserve the three-dimensional configuration of the active site of the native enzyme, such that the activity of the enzyme is preserved.

In certain embodiments of the methods, the MalE fusion protein, His-tag or other suitable recombinant additions are used to enhance expression and/or purification but have preferably been removed from the enzymatically active glucotransferase enzyme when used in the inventive methods.

The methods of the invention can be suitably performed in solution and in any suitable reactor type. The enzyme may in certain embodiments be immobilized on suitable inert media by covalent (e.g. by cross-linking) or non-covalent attachment, such as for continuous operation of the method of the invention. Accordingly, the methods can be operated in e.g moving bed reactors, packed bed reactors, and the like.

The isolated enzyme in the methods of the invention can be used in any concentration suitable in any particular conditions, depending on the nature of substrate, etc. In some embodiments the enzyme is used in a concentration in the reaction medium in the range of about 0.01 to about 20 mg/mL, such as in the range of about 0.05 to about 10 mg/mL, such as in the range of about 0.1 to about 5 mg/mL or in the range of about 0.1 to about 1.0 mg/mL, e.g. about 0.10, about 0.20, about 0.40, about 0.50, about 1.0, or about 2.0 mg/mL. Suitable enzyme concentration may depend on desired reaction time, and amount and concentration of substrate, etc. Substrate concentration can in some embodiments be in the range from about 0.40 to about 50 mg/mL, such as in the range of about 1.0 to about 10 mg/mL.

The methods of the invention can be suitably operated at desired temperature, in certain embodiment the methods are operated at about room temperature (20-25°C) or can in some embodiment be operated at a temperature in the range from about 10°C to about 50°C, such as preferably in the range from about 20 to about 40°C, such as in the range from about 20°C to about 30°C, e.g. at about 25°C or at about 30°C.

### BRIEF DESCRIPTION OF THE FIGURES

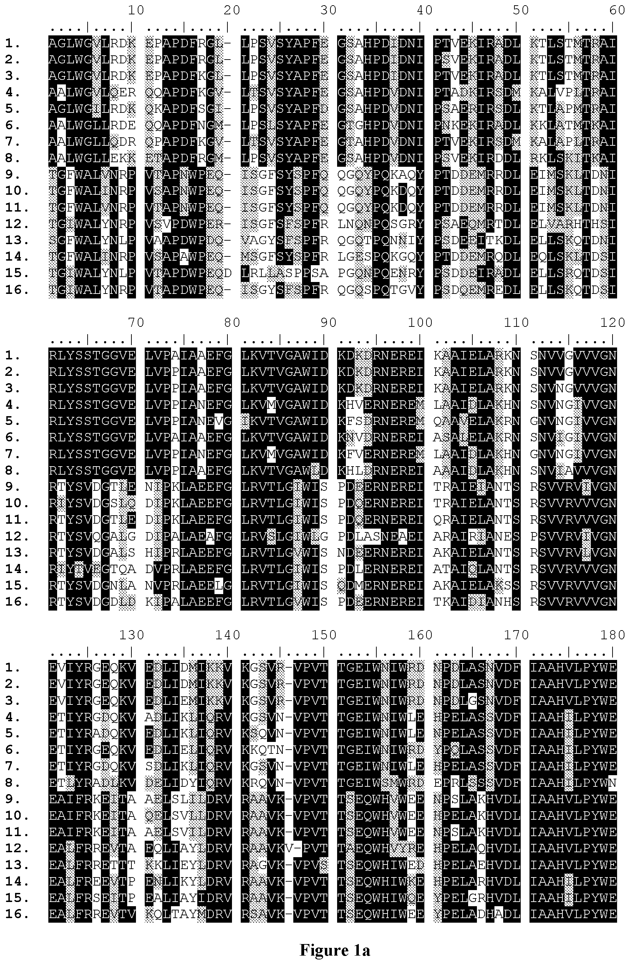

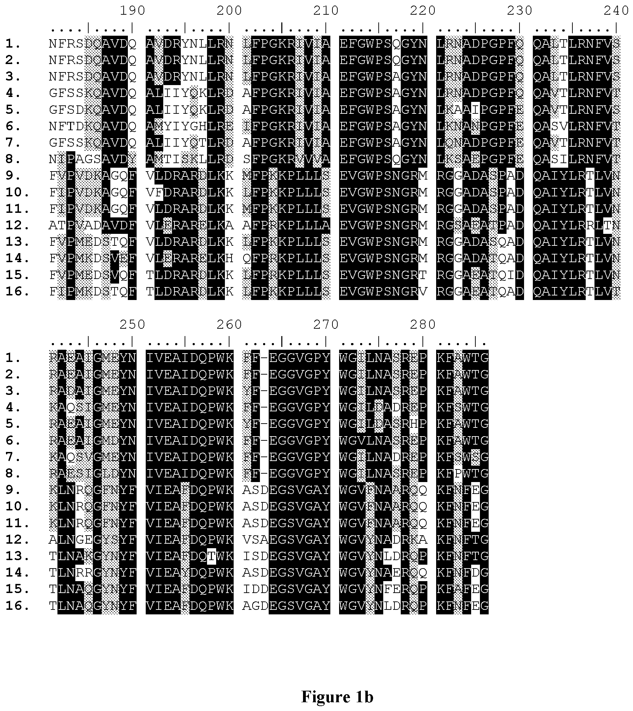

Figure 1 shows amino acid sequence alignment of the GH17 domains including the protein sequences Glt1, Glt3, Glt7 and Glt20. The protein sequences 1-8 are GH17 domains from the taxonomic family Bradyrhizobiace of the Alpha-Proteobacteria and include Glt20 from Bradyrhizobium diazoefficiens (USDA 110). The protein sequences 9-16 include the enzymes Glt1, Glt3 and Glt7 that belong to Gamma-Proteobacteria. Group 1a strains of *B. japonicum* including strain USDA110 have recently been taxonomically redefined as a separate species with the proposed name *Bradyrhizobium diazoefficiens* (Delamuta et al. 2013).
Figure 2 shows the purification and size of the different forms of the cloned Glt20 enzyme run on a 10% SDS gel of Glt20 in crude extract and purification fractions. (1) Protein standard. (2) Sample on amylose column. (3) Sample after amylose column. (4) Sample after ULP reaction. The arrows point to the following bands: (A) Enzyme with MalE domain. (B) MalE domain. (C) Isolated GH17 Enzyme Glt20.
Figure 3 shows TLC of product mixtures (**Pro-Glcₓ**) generated after 24 h incubation of (β1→3)-linked gluco-oligosaccharides (**Lam-Glc₂** - **Lam-Glc₁₀**) with Glt20 enzyme. The figure shows the products from the reaction of Glt20 with β-glucan substrates of different length (DP2-DP10).
Figure 4 shows the structures suggested to be formed from Lam-Glc₅ incubated with Glt20 for 48 h.
Figure 5 shows the 500-MHz ¹H NMR spectrum of **Pro-Glc₁₀,** obtained from **Lam-Glc₆,** recorded in D₂O at 335 K.
Figure 6 shows the structures suggested to be formed from **Lam-Glc₆** incubated with Glt20 for 48 h.
Figure 7 shows total reaction mixtures after 0, 48 and 72 h incubation, when analyzed by MALDI-TOF-MS and NMR-spectroscopy.

### DETAILED DESCRIPTION OF THE INVENTION

Bioinformatic analysis of DNA sequence databases was used to identify multidomain β-glucan branching enzymes. GH17 enzyme domains were isolated, cloned, and expressed in soluble form in E. coli, to investigate their activity. A phylogenetic tree was made from amino acid alignment of selected GH17 sequences from Gamma-Proteobacteria containing Glt1, GL3 and Glt7 and selected sequences from the family sand the *Bradyrhizobiaceae* of Alpha-Proteobacteria. Highly discernable differences are seen between the alpha and gamma-proteobacteria sequences and the *Bradyrhizobiaceae* show high similarity and site specific conservation not found in the GH17 domains from the Gamma-Proteobacteria.Preferred embodiments of the current invention relate to GH17 enzymes from the taxonomic family of *Bradyrhizobiaceae.* The recombinant enzymes used in the examples were produced with attachments that aid their expression and purification, followed by specific removal of the attachments before assaying them for transglycosyl activity. An assay for transglycosyl activity on (β1→3)-linked gluco-oligosaccharides was developed and activity was studied with several analytical methods.

The methods of the invention use glycosyltransferase enzyme of bacterial origin, the term indicating that the enzyme is encoded by a gene originating from bacteria, which may suitably be isolated and transfected in a suitable expression host as described herein.

### EXAMPLE 1

### Sequence analysis of GH17 type I enzymes

This example shows the alignment of amino acid sequences of the GH17 domains including the protein sequences Glt1, Glt3, Glt7 and Glt20. In Figure 1 the protein sequences 1-8 are GH17 domains from the taxonomic family *Bradyrhizobiace* of the Alpha-Proteobacteria and include Glt20 from *Bradyrhizobium diazoefficiens* (USDA 110). As can be seen by the alignment in Figure 1 the protein sequences 1-8 from the taxonomic family *Bradyrhizobiace* are very similar and more closely related than to the protein sequences 9-16, which include the enzymes Glt1, Glt3 and Glt7 that belong to Gamma-Proteobacteria.

The sequences are the following:
1. Glt20-Bradyrhizobium diazoefficiens (USDA-110) (SEQ ID NO: 1)
2. Bradyrhizobium diazoefficiens-(USDA-110) gi|27379725|
3. Bradyrhizobium japonicum (USDA-6 gi|384218756|
4. Nitrobacter hamburgensis(X14) gi|92117324|
5. Rhodopseudomonas palustris (BisB5) gi|91977083|
6. Afipia clevelandensis gi|488799329
7. Nitrobacter sp.Nb-311A gi|497485564|
8. Afipia sp.1NLS2-gi|496697991|
9. Pseudomonas sp.GM79] gi|398239141|
10. Pseudomonas fluorescens-gi|515552181|
11. Pseudomonas sp.45MFCol3.1-gi|518477590|
12. Glt1-Pseudomonas aeruginosa (PAO1)
13. Pseudomonas sp.S9 gi|498172591|
14. Glt3-Pseudomonas putida (KT2440)
15. Glt7-Azotobacter vinelandii (ATCC BAA-1303)
16. Pseudomonas fulva-12-X gi|333901654|

### EXAMPLE 2

### Expression of Glt20 enzyme and activity tests

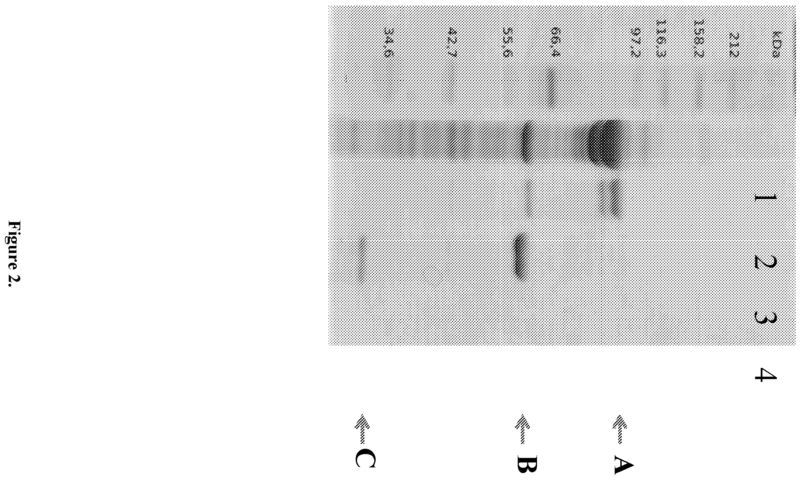

This example demonstrates the production and use of enzymatically active proteins containing the GH17 domain of a Type I enzyme Glt20. An *E. coli* expression vector from Motejadded et al. (2009) was used for the cloning and expression of the glucosyltransferase and to facilitate the purification of the recombinant enzymes. The partial gene encoding the Glt20 protein domain was fused with a gene sequence encoding a maltose binding domain sequence and a His-tag sequence for affinity purification. A *Saccharomyces cerevisiae* Smt3 domain sequence, optimized for the expression in *E. coli*, was located between the maltose binding domain sequence and the cloned gene. Expression of the enzyme was induced with L-Rhamnose.. For the use of the system it was important to cultivate and purify Ulp1 protease which cleaves the fusion protein at the Smt3 site. High yields of soluble protein were achieved with Glt20 (Figure 2).

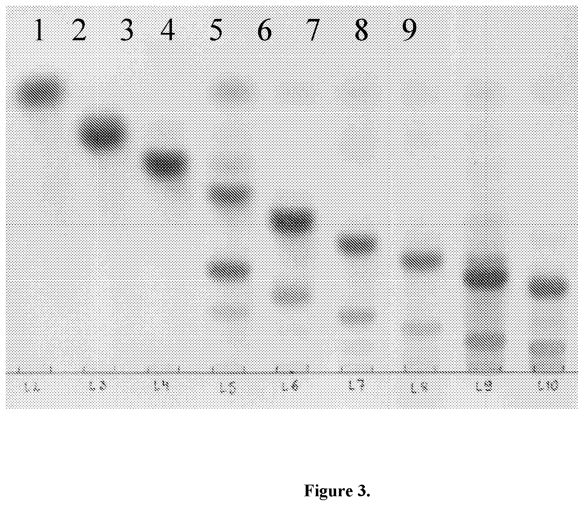

After purification the enzyme was routinely tested at 30°C on -glucan substrates of different length (DP2 to DP10) for 3 days. The reaction progress was monitored by spotting the reaction on TLC. The reaction was as follows: 100 µL substrate DP2 to DP10 (β1→3)-linked oligosaccharides from curdlan (6.25 mg/mL); 30 µL 0.5 M potassium phosphate pH 6.5; 70 µL purified enzyme (1.0 mg/mL) (Figure 3, after 24 h). The TLC is run without oligosaccharide standards. Lane 1 shows incubation with biose, lane 2 with triaose, lane 3 with tetraose, lane 4 with pentaose, lane 5 with hexaose, lane 6 with heptaose, lane 7 with octaose, lane 8 with nonaose, and line 9 with decaose. For DP5-DP10 clear product formation (**Pro-Glcₓ**) at shorter running positions than the starting substrates is seen (see text for explanations).

The activity-screening results for (β1→3)-linked gluco-oligosaccharides **Lam-Glc₅** to **Lam-Glc₁₀** showed that the Glt20 enzyme always releases laminaribiose (DP2) from the reducing end of substrate and adds the remainder to another substrate molecule. TLC bands lower than the substrate indicate that also larger oligosaccharides are formed. The most intensive transfer products formed after 48 h are: from **Lam-Glc₅** (substrate) → **Pro-Gl_{c8}** (product) → **Pro-Glc₁₁** (product) → **Pro-Glc₁₄** (product); from **Lam-Glc₆** → **Pro-Glc₁₀** → **Pro-Glc₁₄** → **Pro-Glc₁₈**; from **Lam-Glc₇** → **Pro-Glc₁₂** → **Pro-Glc₁₇**; from **Lam-Glc₈** → **Pro-Glc₁₄** → **Pro-Glc₂₀**; from **Lam-Glc₉** → **Pro-Glc₁₆** → **Pro-Glc₂₃**; and from **Lam-Glc₁₀** → **Pro-Glc₁₈** → **Pro-Glc₂₆**. According to MALDI-TOF-MS, the amount of the formed larger oligosaccharides after 48 h, e.g. **Pro-Glc₈** and **Pro-Glc₁₁** in case of **Lam-Glc₅** was estimated to be about 60% of the total sample amount.

### EXAMPLE 3

### General strategy for incubation of laminari-oligosaccharides (Lam-Glcₓ) with the Glt20 enzyme and analysis of the products formed

(β1-3)-Gluco-oligosaccharides(-alditols) (**Lam-Glc₂** - **Lam-Glc₁₀**) were incubated with the Glt20 enzyme in 0.5 M phosphate buffer, pH 6.5, at 30 °C. The progress of the reaction (0-72 h) was followed by analyzing aliquots on TLC (Merck Kieselgel 60 F254 sheets; butanol:acetic acid:water = 2:1:1; orcinol/sulfuric acid staining). After 24 h, 48 h and 72 h of incubation, the product(s) were isolated by gel-permeation chromatography (GPC) on a Bio-Gel P-2 column (90 x 1 cm), eluted with 10 mM ammonium bicarbonate, at a flow rate of 12 mL/h. In the high-mass-elution region, fractions of 5 min were collected and analyzed by TLC. Fractions containing oligosaccharides with increased DP (compared to the substrate oligosaccharide) were investigated by MALDI-TOF mass spectrometry and 1D/2D Nuclear Magnetic Resonance (NMR) spectroscopy.

### Isolation and characterization of reaction products from the incubation of Lam-Glc₅

The substrate **Lam-Glc₅** was incubated for 24 h with the cloned Glt20 bacterial enzyme. The TLC-screening showed that Glt20 cleaves (β1→3) linkages in the substrate and releases a biose and a tetraose as the main hydrolysis products (Figure 3). Bands lower than the substrate indicate that also larger oligosaccharides are formed. The MALDI-TOF mass spectrum showed major molecular ions [M+K]⁺ at *m*/*z* 867.2, corresponding to **Lam-Glc₅**, and *m*/*z* 1353.5 and *m*/*z* 1839.8, corresponding to **Pro-Glc₈** and **Pro-Glc₁₁**, respectively. Oligosaccharide **Lam-Glc₅** (1000 µL; 6.25 mg/mL) was incubated for 48 h with Glt20 (700 µL; 1.0 mg/mL) in 0.5 M KH₂PO₄ / K₂HPO₄ buffer (300 µL), pH 6.5, at 30 °C. The mixture of oligosaccharides was fractionated on Bio-Gel P-2, yielding fractions **I** to **X**, which were screened by TLC and MALDI-TOF-MS. The MALDI-TOF mass spectrum of fraction **V** showed one major sodiated molecular ion [M+Na]⁺ at *m*/*z* 1337.4, corresponding to **Pro-Glc₈** and very minor peak intensities (<5%) for **Pro-Glc₇** (*m*/*z* 1175.1) and **Pro-Glc₉** (*m*/*z* 1449.6). The MALDI-TOF mass spectrum of fraction **III** showed one major sodiated molecular ion [M+Na]⁺ at *m*/*z* 1823.9, corresponding to **Pro-Glc₁₁** and very minor peak intensities (<10%) for **Pro-Glc₁₀** (*m*/*z* 1661.2) and **Pro-Glc₁₂** (*m*/*z* 1965.3).

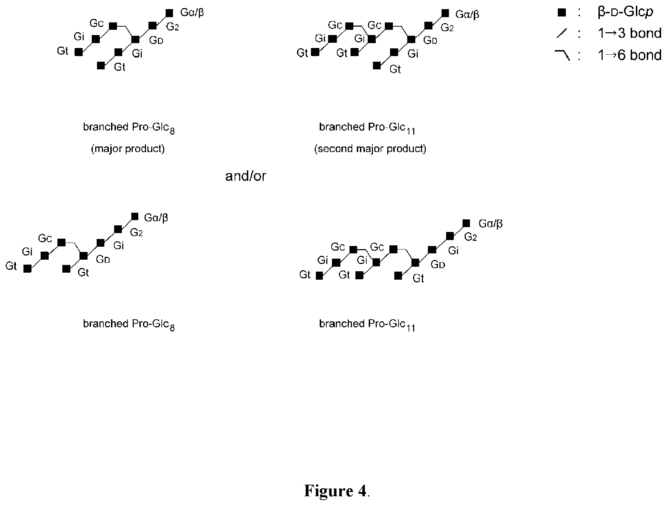

Based on combined MS, 1D/2D NMR, and enzymatic degradation data, and the hypothesis that **Pro-Glc₈** and **Pro-Glc₁₁** are synthesized by a transfer of the triaose β-D-Glc*p-*(1→3)-β-D-Gl*cp*-(1→3)-β-D-Glc*p*- to the substrate **Lam-Glc₅**, the following structures shown in Figure 4, are suggested to be present in fraction **V** and in fraction **III**.

### Isolation and characterization of reaction products from the incubation of Lam-Glc₆

The substrate **Lam-Glc₆** was incubated for 24 h with the cloned Glt20 bacterial enzyme. The TLC-screening showed that Glt20 cleaves (1→3) linkages in the substrate and releases a tetraose, showing biose as the main hydrolysis product (Figure 3). Bands lower than the substrate indicate that also larger oligosaccharides are formed. The MALDI-TOF mass spectrum showed a major molecular ion [M+K]⁺ at *m*/*z* 1029.2 corresponding to **Lam-Glc₆** and *m*/*z* 1677.5, corresponding to **Pro-Glc₁₀**. Very minor molecular ions [M+K]⁺ at *m*/*z* 2325.8 and *m*/*z* 2974.0, corresponding to **Pro-Glc₁₄** and **Pro-Glc₁₈**, respectively, were also present.

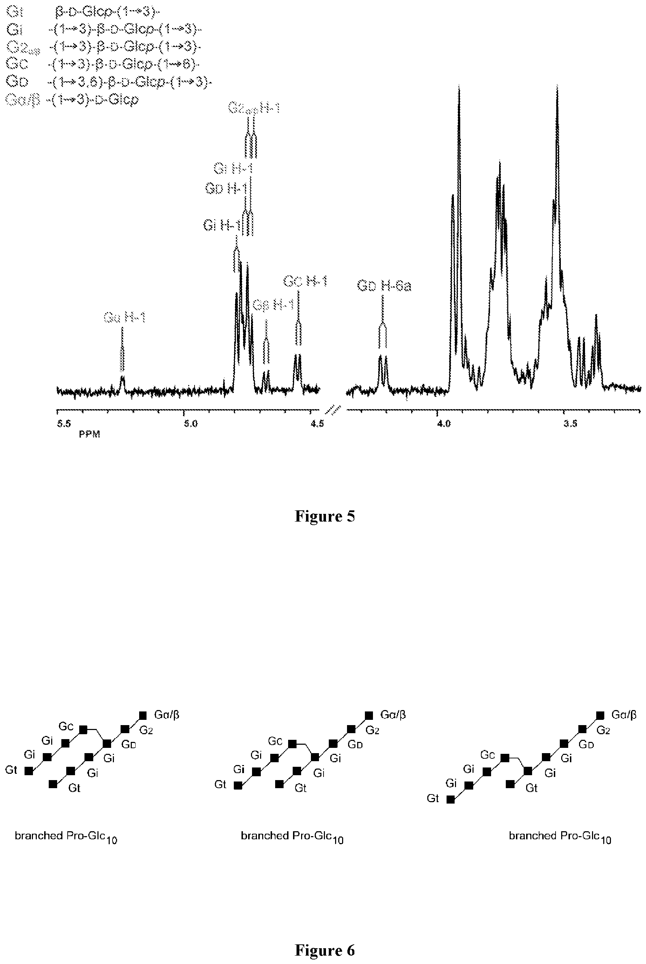

Oligosaccharide **Lam-Glc₆** (1000 µL; 6.25 mg/mL) was incubated for 48 h with Glt20 (700 µL; 1.0 mg/mL) in 0.5 M KH₂PO₄ / K₂HPO₄ buffer (300 µL), pH 6.5, at 30 °C. The mixture of oligosaccharides was fractionated on Bio-Gel P-2, yielding fractions **I** to **XII**, which were screened by TLC and MALDI-TOF-MS. The MALDI-TOF mass spectrum of fraction IV showed one major sodiated molecular ion [M+Na]⁺ at *m*/*z* 1661.5, corresponding to **Pro-Glc₁₀** and very minor peak intensities (<20%) for **Pro-Glc₉** (*m*/*z* 1499.4) and **Pro-Glc₁₁** (*m*/*z* 1823.7). In the 1D ¹H NMR spectrum (Figure 5) of fraction **IV** the same anomeric protons of **Gi**, **Gt**, **G2**, **Gα/β**, **GC**, **GD** were present as in the products formed from **Lam-Glc₅**. Complete assignment indicated that **Pro-Glc₁₀** contains one single branching point.

Based on combined MS and 1D/2D NMR data, and the hypothesis that the major products **Pro-Glc₁₀** and **Pro-Glc₁₄** are synthesized by the transfer of a tetraose, β-D-Gl*cp-*(1→3)-β-D-Glc*p*-(1→3)-β-D-Glc*p*-(1→3)-β-D-Glc*p*-, to the substrate **Lam-Glc₆**, the following structures for **Pro-Glc₁₀** are shown in Figure 6.

### The key properties of the Glt20 enzyme determined

The Glt20 enzyme is not active on **Lam-Glc_{2/3/4}**. However, Glt20 cleaves a biose from the reducing end of the substrate (DP>4) donor and add the remainder oligosaccharide part again to substrate. The optimal incubation time is about 48 h to get oligosaccharides with DP>DPsubstrate, together with a minimal amount of hydrolysis products.

For **Lam-Glc₅** as substrate, the main reaction products are **Pro-Glc₈** and **Pro-Glc₁₁**, together with a minor amount of **Pro-Glc₁₄**, indicating the subsequent transfer of triaose. Initially formed products act again as acceptor.

For **Lam-Glc₆** as substrate, the main reaction product is **Pro-Glc₁₀**. Additionally, **Pro-Glc₁₄** and **Pro-Glc₁₈** are present in minor amounts. Here, there is a subsequent transfer of a tetraose. **Pro-Glc₅**, **Pro-Glc₇** and **Pro-Glc₉** are observed as minor hydrolysis products.

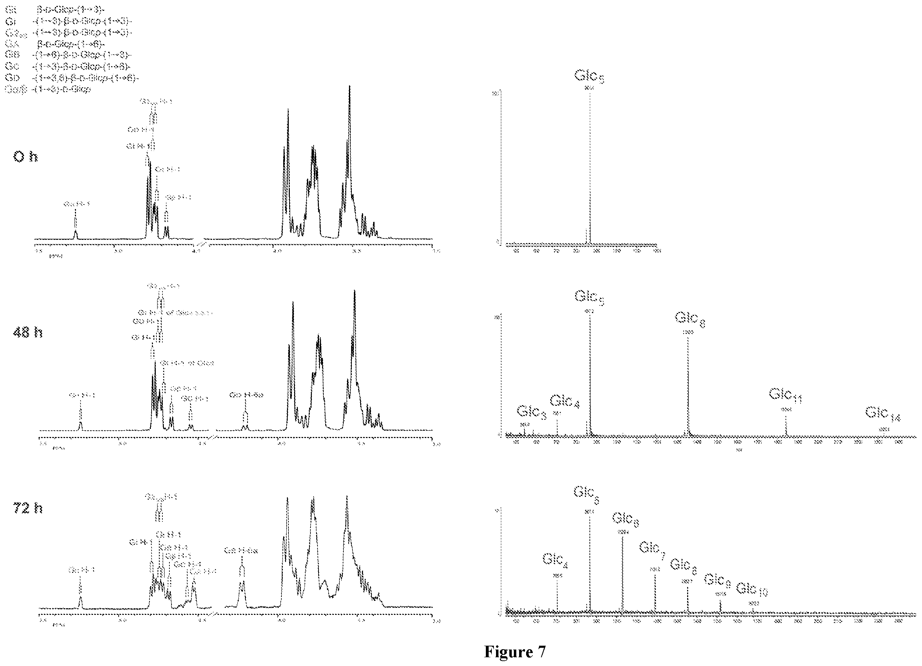

The ¹H NMR spectra of the isolated high-mass fractions of both substrates (**Lam-Glc₅** and **Lam-Glc₆**) show that branching takes place up to 48 h of incubation. It should be noted that after 72 h of incubation, when all substrate is consumed, the hydrolysis activity occurs and the branched products are being degraded into linear products (containing internal (β1→6) linkages) (see Figure 7). This indicates that the transferase activity, resulting in products built up from oligomeric (β1→3) glucan fragments, coupled via (β1→6) branching on oligomeric (β1→3) glucan fragments (multiple branching), is highly preferred over the hydrolysis reaction, which only becomes significant after the depletion of the donor substrate. It has to be noted that several structures are possible for a product of a certain DP. Analysis of the remaining structures after 72 h incubation showed that from a starting material that contained no (β1→6) linkages, at the end up to 26% of all linkages were now (β1→6). Since in the starting material of **Lam-Glc₅** there are four (β1→3) linkages, it means that one is left in the leaving laminari-biose and two are in the transferred laminari-triose. This therefore means that about 100% of the substrate had been cleaved and transferred into a branched product. This is therefore the highest ratio of introduced branches made by any known glucan transferase.

The activity of the Glt20 enzyme starting from the reducing end of the substrate, was confirmed by the fact that the enzyme was not active on oligosaccharide-alditols.

### REFERENCES.

B 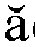 dulescu, M., Apetrei, N. S., Lupu, A. R., Cremer, L., Szegli, G., Moscovici, M., Mocanu, G., Mihai, D., 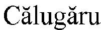 , A. (2009). Curdlan derivatives able to enhance cytostatic drugs activity on tumor cells. Roumanian Archives of Microbiology and Immunology, 68, 201-206.
Bhagwat, A. A., and D. L. Keister. 1995. Site-directed mutagenesis of the b-(1,3),b-(1,6)-D-glucan synthesis locus of Bradyrhizobium japonicum. Mol.Plant-Microbe Interact. 8:366-370.
Bhagwat, A. A., Mithöfer, A., Pfeffer, P. E., Kraus, C., Spickers, N., Hotchkiss, A., Ebel, J., et al. (1999). Further studies of the role of cyclic beta-glucans in symbiosis. An NdvC mutant of Bradyrhizobium japonicum synthesizes cyclodecakis-(1-->3)-beta-glucosyl. Plant Physiology, 119(3), 1057-1064.
Delamuta J.R., Ribeiro R.A., Ormeño-Orrillo E., Melo I.S., Martinez-Romero E., Hungria M. (2013). Polyphasic evidence supporting the reclassification of Bradyrhizobium japonicum group Ia strains as Bradyrhizobium diazoefficiens sp. nov. Int J Syst Evol Microbiol. 63, 3342-3351.
Gastebois, A., Mouyna, I., Simenel, C., Clavaud, C., Coddeville, B., Delepierre, M., Latgé, J., Fontaine, T. (2010). Characterization of a new β(1-3)-glucan branching activity of Aspergillus fumigatus. Journal of Biological Chemistry, 285, 2386-2396.
Hreggvidsson, G. O., Dobruchowska, J. M., Fridjonsson, O. H., Jonsson, J. O., Gerwig G. J., Aevarsson, A., Kristjansson J. K., Curti, D., Redgwell, R. R., Hansen, C.-E., Kamerling, J. P., and Debeche-Boukhit, T. (2011). Exploring novel non-Leloir β-glucosyltransferases from proteobacteria for modifying linear (β1→3)-linked gluco-oligosaccharide chains. Glycobiology, 21, 304-328/664-688.
Jon O. Jonsson (2010). β-Glucan transferases of Family GH17 from Proteobacteria. M.Sc. Thesis. University of Iceland.
McIntosh, M., Stone, B. A., Stanisich, V. A. (2005). Curdlan and other bacterial (1→3)-β-D-glucans. Applied Microbiology and Biotechnology, 68, 163-173.
Motejadded, H., Altenbuchner, J. (2009). Construction of a dual-tag system for gene expression, protein affinity purification and fusion protein processing. Biotechnology Letters, 31, 543-549.

## Claims

1. A method for transglycosylation of β-glucan oligo- and/or polysaccharides for making branched oligo- and/or polysaccharides, comprising:
- providing an isolated enzyme of bacterial origin, wherein said isolated enzyme belongs to glycosyl hydrolase family GH17, and comprises an amino acid sequence having at least 75% sequence identity to SEQ ID NO:1, said enzyme having glycosyltransferase activity on β-glucan oligo- and/or polysaccharides, such that it cleaves a donor β-glucan oligo- or polysaccharide and transfers a part of said β-glucan oligo- or polysaccharide to internal glucose unit of an additional β-glucan oligo- or polysaccharide acting as acceptor to covalently bind said part to the acceptor through a (β1→6) linkage,
- bringing the enzyme in contact with a substrate comprising beta-glucan oligo- or - polysaccharides with chains comprising five or more glucose units, and
- forming a covalently linked branch comprising three or more glucose units from a donor β-glucan oligo- or polysaccharide, on an acceptor oligo- or polysaccharide through a (β1→6) linkage.

2. The method of Claim 1 wherein said isolated enzyme of bacterial origin catalyses a transglycosylation reaction such that it releases two glucose units from the reducing end of said donor β-glucan oligo- or polysaccharide and transfers the remaining non-reducing end to an internal glucose unit of said additional β-glucan oligo- or polysaccharide thus forming a covalently linked branch comprising three or more glucose units on said acceptor polysaccharide.

3. The method of Claim 1 wherein said isolated enzyme transfers said part of said donor β-glucan oligo- or polysaccharide to the third glucose residue from the reducing end of said acceptor β-glucan oligo- or polysaccharide.

4. The method of Claim 1 wherein said isolated enzyme of bacterial origin has preferential transferase activity over hydrolase activity in the presence of reactive acceptor β-glucan oligo- or polysaccharide.

5. The method of Claim 1 wherein a covalently linked branch is formed on another covalently linked branch in an acceptor β-glucan oligo- or polysaccharide thus forming a product oligo- or polysaccharide with branch on branch.

6. The method of Claim 1 wherein the donor and/or the acceptor oligo- or polysaccharide β-glucan comprises any oligo- or polysaccharide from the group consisting of yeast β-glucan, laminarin, lichenan, curdlan and barley β-glucan.

7. The method of Claim 1 wherein the sequence of said isolated enzyme is from a bacterial strain from the taxonomic family of *Bradyrhizobiaceae.*

8. The method of Claim 1 wherein the sequence of said isolated enzyme is from a bacterial strain from the taxonomic genus of *Bradyrhizobium.*

9. The method of Claim 1 wherein the sequence of said isolated enzyme is from a bacterial origin comprises bacterial strains belonging to the species *Bradyrhizobium japonicum* or Bradyrhizobium diazoefficiens.

10. The method of any of claims 1-9 wherein the produced branched oligo- and/or polysaccharides have biological activity.

11. The method of claim 10 where the biological activity comprises immune activity such as anti-tumoral, anti-bacterial, anti-viral or anti-fungal activity.

12. The method of claim 10 for the production of bio-active β-glucan molecules for cosmetic purposes.

13. The method of claim 11 for the production of bio-active β-glucan molecules for medical/pharmaceutical use.

14. The method of claim 13 for the production of bio-active β-glucan molecules as constituents of drugs.

## Patentansprüche

1. Verfahren zur Transglycosylierung von β-Glucan-oligo- und/oder -polysacchariden zur Herstellung von verzweigten Oligo- und/oder Polysacchariden, das Folgendes umfasst:
- Bereitstellen eines isolierten Enzyms bakteriellen Ursprungs, wobei das isolierte Enzym zur Glycosylhydrolasefamilie GH17 gehört und eine Aminosäuresequenz mit mindestens 75% Sequenzidentität zu SEQ ID NO: 1 umfasst, wobei das Enzym so eine Glycosyltransferaseaktivität auf β-Glucan-oligo- und/oder -polysaccharide ausübt, dass es Donor-β-Glucan-oligo- oder -polysaccharide spaltet und einen Teil des β-Glucan-oligo- oder -polysaccharids auf eine interne Glucoseeinheit eines zusätzlichen β-Glucan-oligo- oder -polysaccharids, das als Akzeptor dient, überträgt, so dass dieser Teil über eine (β1→6)-Bindung kovalent an den Akzeptor gebunden wird,
- Inkontaktbringen des Enzyms mit einem Substrat, das beta-Glucan-oligo- und/oder -polysaccharide mit Ketten mit fünf oder mehr Glucoseeinheiten umfasst, und
- Bilden einer kovalent gebundenen Verzweigung mit drei oder mehr Glucoseeinheiten von einem Donor-β-Glucan-oligo- oder -polysaccharid an einem Akzeptor-oligo- oder -polysaccharid durch eine (β1→6-Bindung).

2. Verfahren nach Anspruch 1, wobei das isolierte Enzym bakteriellen Ursprungs eine Transglycosylierungsreaktion so katalysiert, dass es zwei Glucoseeinheiten vom reduzierenden Ende des Donor-β-Glucan-oligo- oder -polysaccharids freisetzt und das verbleibende nichtreduzierende Ende an eine interne Glucoseeinheit des zusätzlichen β-Glucan-oligo- oder -polysaccharids überträgt, so dass eine kovalent gebundene Verzweigung mit drei oder mehr Glucoseeinheiten an dem Akzeptorpolysaccharid entsteht.

3. Verfahren nach Anspruch 1, wobei das isolierte Enzym den Teil des Donor-β-Glucan-oligo- oder -polysaccharids auf dem dritten Glucoserest vom reduzierenden Ende des Akzeptor-β-Glucan-oligo- oder -polysaccharids überträgt.

4. Verfahren nach Anspruch 1, wobei das isolierte Enzym bakteriellen Ursprungs in Gegenwart von reaktionsfähigem Akzeptor-β-Glucan-oligo- oder -polysaccharid bevorzugte Transferaseaktivität im Vergleich zu Hydrolaseaktivität aufweist.

5. Verfahren nach Anspruch 1, wobei eine kovalent gebundene Verzweigung an einer anderen kovalent gebundenen Verzweigung in einem Akzeptor-β-Glucan-oligo- oder -polysaccharid gebildet wird, wodurch ein Produkt-oligo- oder -polysaccharid mit einer verzweigten Verzweigung entsteht.

6. Verfahren nach Anspruch 1, wobei das Donor- und/oder das Akzeptor-oligo- oder -polysaccharid-β-Glucan ein beliebiges Oligo- oder Polysaccharid aus der Gruppe bestehend aus Hefe-β-Glucan, Laminarin, Lichenan, Curdlan und Gersten-β-Glucan umfasst.

7. Verfahren nach Anspruch 1, wobei die Sequenz des isolierten Enzyms von einem Bakterienstamm aus der taxonomischen Familie der *Bradyrhizobiaceae* stammt.

8. Verfahren nach Anspruch 1, wobei die Sequenz des isolierten Enzyms von einem Bakterienstamm aus der taxonomischen Gattung *Bradyrhizobium* stammt.

9. Verfahren nach Anspruch 1, wobei die Sequenz des isolierten Enzyms von einem bakteriellen Ursprung stammt, der Bakterienstämme umfasst, die zu der Art *Bradyrhizobium japonicum* oder Bradyrhizobium diazoefficiens gehört.

10. Verfahren nach einem der Ansprüche 1-9, wobei die erzeugten verzweigten Oligo- und/oder Polysaccharide biologische Wirksamkeit aufweisen.

11. Verfahren nach Anspruch 10, wobei die biologische Aktivität Immunaktivität wie antitumorale, antibakterielle, antivirale oder antifungale Aktivität umfasst.

12. Verfahren nach Anspruch 10 für die Erzeugung von biologisch aktiven β-Glucanmolekülen für kosmetische Zwecke.

13. Verfahren nach Anspruch 11 für die Erzeugung von biologisch aktiven β-Glucanmolekülen für die medizinische/pharmazeutische Verwendung.

14. Verfahren nach Anspruch 13 für die Erzeugung von biologisch aktiven β-Glucanmolekülen als Arzneistoffbestandteile.

## Revendications

1. Procédé de transglycosylation d'oligo- et/ou de polysaccharides de β-glucane destinés à élaborer des oligo- et/ou polysaccharides ramifiés, comprenant les étapes consistant à :
- fournir une enzyme isolée d'origine bactérienne, ladite enzyme isolée appartenant à la famille des glycosyl hydrolases GH17 et comprenant une séquence d'acides aminés ayant une identité de séquence, d'au moins 75%, avec la SEQ ID n° : 1, ladite enzyme ayant une activité de glycosyltransférase sur des oligo- et/ou polysaccharides de β-glucane, de sorte qu'elle coupe un oligo- ou polysaccharide de β-glucane donneur et transfère une partie dudit oligo- ou polysaccharide de β-glucane vers une unité de glucose interne d'un oligo- ou polysaccharide de β-glucane supplémentaire agissant comme accepteur, pour lier par covalence ladite partie à l'accepteur au moyen d'une liaison (β1→6),
- amener l'enzyme au contact d'un substrat, comprenant des oligo- ou polysaccharides de bêta-glucane avec des chaînes qui comprennent cinq unités de glucose ou plus, et
- former une ramification liée par covalence, qui comprend trois unités de glucose ou plus, à partir d'un oligo- ou polysaccharide de β-glucane donneur sur un oligo- ou polysaccharide accepteur, au moyen d'une liaison (β1→6).

2. Procédé selon la revendication 1, dans lequel ladite enzyme isolée d'origine bactérienne catalyse une réaction de transglycosylation, de sorte qu'elle libère deux unités de glucose à partir de l'extrémité réductrice dudit oligo- ou polysaccharide de β-glucane donneur et transfère l'extrémité non réductrice restante vers une unité de glucose interne dudit oligo- ou polysaccharide de β-glucane supplémentaire, en formant ainsi une ramification liée par covalence, qui comprend trois unités de glucose ou plus, sur ledit polysaccharide accepteur.

3. Procédé selon la revendication 1, dans lequel ladite enzyme isolée transfère ladite partie dudit oligo- ou polysaccharide de β-glucane donneur vers le troisième résidu de glucose à partir de l'extrémité réductrice dudit oligo- ou polysaccharide de β-glucane accepteur.

4. Procédé selon la revendication 1, dans lequel ladite enzyme isolée d'origine bactérienne a une activité préférentielle de transférase par rapport à l'activité d'hydrolase en présence d'un oligo- ou polysaccharide de β-glucane accepteur réactif.

5. Procédé selon la revendication 1, dans lequel une ramification liée par covalence est formée sur une autre ramification liée par covalence dans un oligo- ou polysaccharide de β-glucane accepteur, en formant ainsi un oligo- ou polysaccharide produit avec une ramification sur une ramification.

6. Procédé selon la revendication 1, dans lequel l'oligo- ou le polysaccharide de β-glucane donneur et/ou accepteur comprend n'importe quel oligo- ou polysaccharide provenant du groupe constitué par un β-glucane de levure, la laminarine, le lichénane, le curdlan et un β-glucane de l'orge.

7. Procédé selon la revendication 1, dans lequel la séquence de ladite enzyme isolée provient d'une souche bactérienne de la famille taxonomique des *Bradyrhizobiacées.*

8. Procédé selon la revendication 1, dans lequel la séquence de ladite enzyme isolée provient d'une souche bactérienne du genre taxonomique *Bradyrhizobium.*

9. Procédé selon la revendication 1, dans lequel la séquence de ladite enzyme isolée est d'origine bactérienne qui comprend des souches bactériennes appartenant à l'espèce *Bradyrhizobium japonicum* ou Bradyrhizobium diazoefficiens.

10. Procédé selon l'une quelconque des revendications 1 à 9, dans lequel les oligo- et/ou polysaccharides ramifiés produits ont une activité biologique.

11. Procédé, selon la revendication 10, où l'activité biologique comprend une activité immunologique telle qu'une activité anti-tumorale, antibactérienne, antivirale ou antifongique.

12. Procédé, selon la revendication 10, destiné à la production de molécules bioactives de β-glucane à des fins cosmétiques.

13. Procédé, selon la revendication 11, destiné à la production de molécules bioactives de β-glucane pour une utilisation médicale/pharmaceutique.

14. Procédé, selon la revendication 13, destiné à la production de molécules bioactives de β-glucane comme constituants de médicaments.
